# FASCICULE DE BREVET EUROPEEN

(11) **EP 0 620 724 B1**
(45) Date de publication et mention de la délivrance du brevet: **25.09.1996**
(21) Numéro de dépôt: 93904074.7
(22) Date de dépôt: 13.01.1993
(51) Int. Cl.: A61K 7/16

(54) **COMPOSITION ET PROCEDE D'HYGIENE BUCCALE**
PRÄPARAT UND VERFAHREN ZUR ORALEN HYGIENE
ORAL HYGIENE COMPOSITION AND PROCESS

(30) Priorité: 15.01.1992 FR 9200352
(43) Date de publication de la demande: 26.10.1994
(73) Titulaire: L'OREAL, F-75008 Paris (FR)
(72) Inventeur: ASCIONE, Jean-Marc, F-75018 Paris (FR); FORESTIER, Serge, F-77410 Claye-Souilly (FR)
(74) Mandataire: Casalonga, Axel
(86) Numéro de dépôt international: FR9300028
(87) Numéro de publication internationale: WO9313747

(56) Documents cités:
- US-A- 4 992 258

## Description

La présente invention concerne des compositions à usage buccal destinées à éviter la formation de taches sur les dents et l'adhésion des. substances exogènes, ainsi qu'un procédé d'hygiène buccale les mettant en oeuvre.

La formation de taches sur les dents pose un problème esthétique à beaucoup d'individus et notamment ceux qui sont sujets à la formation de taches de goudron, de tartre et de taches dues à l'alimentation qu'ils ingèrent, ceux qui fument ou consomment du café ou du thé.

L'adsorption et la rétention de substances alimentaires contribuent pour une grande part à la formation des taches sur les dents, qu'elles soient naturelles ou artificielles, qui provient en partie de l'adhésion de substances exogènes. Les substances principalement impliquées dans la formation des taches sont notamment le thé, le café, les vins, les fruits rouges et le tabac.

La formation de taches peut également être provoquée par des agents antibactériens anti-plaque : ainsi, on a noté que des biguanidohexanes tels que la Chlorhexidine et l'Alexidine et des sels d'ammonium quaternaires tels que le chlorure de Benzéthonium ou le chlorure de Cétylpyridinium pouvaient être à l'origine de la formation de taches.

Dans les brevets US 4.224.309, US 4.118.474 et US 4.118.473, on a déjà proposé d'utiliser des additifs tels que l'acide 2-phosphonobutane-1,2,4-tricarboxylique, l'acide phosphonoacétique ou l'acide iminodiacétique N-méthylène phosphonique dans le but de réduire la formation de taches sur les dents, engendrée par l'utilisation continuelle d'antibactériens.

Par ailleurs, dans le document EP 373.688, on a décrit l'utilisation d'organopolysiloxanes pour prévenir l'adhésion des goudrons, des taches, du tartre et des particules alimentaires sur les dents.

Enfin, certains polymères anioniques synthétiques linéaires, polycarboxylates, ont été décrits pour une application buccale, notamment, en association avec une source d'ions fluorures, pour favoriser l'action de certains anti-tartres aux polyphosphates alcalins en empêchant leur hydrolyse enzymatique par la salive. Parmi ces polymères, on peut citer les carboxyvinyliques réticulés, connus sous la dénomination commerciale "CARBOPOL" (CARBOPOL 934, CARBOPOL 940, CARBOPOL 941) de BF GOODRICH, les copolymères de l'anhydride maléique et de méthylvinyléther ayant un poids moléculaire moyen allant de 30.000 à 1.000.000, et de préférence 30.000 à 500.000 et disponibles sous la dénomination commerciale "GANTREZ" et plus particulièrement le "GANTREZ S-97 PHARMACEUTICAL GRADE (qui a un poids moléculaire moyen de 70.000) de GAF CORPORATION, ainsi que des copolymères acétate de vinyle/acide crotonique dont certains sont connus sous la dénomination commerciale "ARISTOFLEX A" de HOECHST.

De façon surprenante, la demanderesse a maintenant mis en évidence certains polymères synthétiques hydrocarbonés de structure linéaire ou réticulée et porteurs de groupes carboxylate et de groupes hydroxyméthyle, permettant d'éviter la formation de taches sur les dents de façon efficace. L'activité anti-taches a notamment été mise en évidence in vitro par mesure colorimétrique de pastilles de poudre d'hydroxyapatite (HAP) traitée avec une solution de polymère en comparaison avec des pastilles de poudre d'HAP non-traitée. De plus, les compositions à usage buccal comportant ces polymères ont non seulement une action préventive sur la formation de taches évoquée ci-dessus, à savoir par rétention et/ou adsorption des substances alimentaires, mais également sur le brunissement des dents naturelles ou artificielles engendré par la présence des agents antibactériens anti-plaques connus. Elles permettent aussi d'éviter de façon efficace l'adhésion de substances exogènes sur les dents et présentent une activité préventive sur l'apparition de la plaque dentaire et du tartre. Elles peuvent ainsi contribuer de façon générale à l'hygiène buccale.

Ainsi, la présente invention concerne l'utilisation, pour l'hygiène buccale, d'une composition comportant au moins un polymère hydrocarboné, à structure linéaire ou réticulée, constitué principalement de motifs de formule : 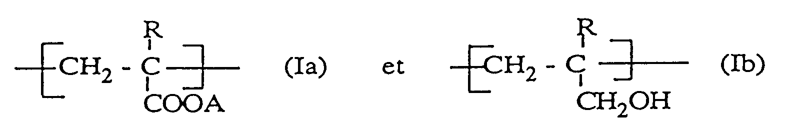 dans lesquelles :
R représente H ou CH₂OH,
A représente un métal alcalin ou un groupement sel d'ammonium, d'amine ou d'alcanolamine, la fréquence moyenne des motifs correspondant à un rapport carboxylate/hydroxyméthyle supérieur à 0,5 dans le polymère, et
   un véhicule physiologiquement acceptable.

Les motifs de formules (Ia) et (Ib) peuvent être disposés dans un ordre quelconque et la fréquence moyenne des motifs dans le polymère correspond à un rapport carboxylate/hydroxyméthyle de préférence compris entre 1,1 et 16 et de façon plus préférentielle entre 2 et 9. Le degré de polymérisation moyen est de 3 à 1000 et de préférence de 50 à 600.

Par usage buccal, on entend que l'on n'ingère pas les produits, mais qu'ils peuvent être retenus dans la cavité buccale pendant une durée suffisante pour permettre un contact avec toute la surface dentaire.

Les polymères incorporés selon l'invention aux compositions à usage buccal sont notamment décrits dans le brevet FR 2.334.695. Selon ce document, il est prévu d'utiliser ces polymères dans des produits à usage externe, produits de soins et de protection de la peau. Ils sont en général sous forme de poudre. Il s'agit de polymères linéaires ou réticulés constitués principalement des motifs de formule (I) ci-dessus où A représente en particulier un métal alcalin comme le sodium, ou un groupement sel d'ammonium, d'amine ou d'alcanolamine.

Parmi ces polymères, on préfère selon l'invention ceux pour lesquels le carboxylate est un carboxylate de sodium. Ainsi, on peut utiliser par exemple les polymères connus sous les noms de "HYDAGEN F" ou "HYDAGEN FN".

Préférentiellement, la composition à usage buccal selon l'invention renferme une quantité efficace de polymères connus sous le nom commercial de "HYDAGEN FN", qui est un copolymère acrylate de sodium/vinylcarbinol.

Les compositions selon l'invention renfermant au moins un polymère constitué de motifs de formules (Ia) et (Ib) peuvent se présenter sous diverses formes et notamment sous forme de sprays, de mousses, de bains de bouche, de gargarismes, de poudres, comprimés ou granulés dentaires, de gommes à mâcher, de gel dentaire, ou de pâtes ou gels dentifrices, le véhicule étant choisi selon la forme désirée.

Les compositions à usage buccal selon l'invention peuvent donc contenir, outre le ou les polymères constitués de motifs de formules (Ia) et (Ib), à titre de véhicule ou pour leur activité propre, des ingrédients habituellement utilisés dans les produits à usage buccal sous la forme correspondante.

Ces compositions sont préparées selon les procédés usuels correspondants aux véhicules choisis. Le véhicule physiologiquement acceptable peut être de différente nature selon la forme choisie pour la composition : solution aqueuse, solution hydroalcoolique épaissie ou non, gomme, excipient pâteux ou solide ...

Dans ces compositions, les polymères selon l'invention définis ci-dessus sont présents seuls ou en mélange, dans des quantités efficaces, c'est-à-dire à des concentrations pondérales comprises entre 0,05 et 10%, et de préférence entre 0,1 et 5% en poids par rapport au poids total de la composition.

Selon les formes désirées, elles peuvent contenir en particulier au moins un agent de polissage dans des proportions allant jusqu'à 95%. Les agents de polissages sont des abrasifs qui peuvent être d'origine minérale ou organique. Leur nature peut différer selon le véhicule utilisé pour la forme choisie, comme cela apparaîtra ci-dessous.

De plus, dans certaines formes, les compositions peuvent contenir un ou plusieurs agent(s) tensio-actif(s) suffisamment stables et moussants. Les agents tensio-actifs utilisables peuvent être de nature anionique, amphotère, zwitterionique, cationique ou non-ionique, et on utilise de préférence des tensio-actifs anioniques ou non-ioniques.

De façon générale, les tensio-actifs peuvent être présents dans une gamme pondérale allant jusqu'à 20% par rapport à la composition et particulièrement dans la gamme de 0,5 à 5%.

En outre, les compositions selon l'invention peuvent contenir d'autres agents actifs pour l'hygiène buccale et notamment des agents connus pour détruire la mauvaise haleine, tels que par exemple les cyclodextrines ou des composés du zinc qui sont des sels minéraux ou organiques tels que par exemple les halogénures de zinc, l'acétate de zinc, le citrate de zinc ou le fluorure de zinc.

Par ailleurs, elles peuvent contenir d'autres agents, usuels dans les compositions buccales, tels que les agents de cohésion, les agents épaississants, les agents antibiotiques, les agents édulcorants, humectants ou rafraîchissants, les agents conservateurs, les agents sucrants, les colorants, les arômes, les substances et agents aromatisants ou de sapidité, les agents peptisants, les agents plastifiants, les agents antibactériens ou bactéricides, des vitamines, les agents anticaries, les agents antitartre, les cicatrisants, les vasomoteurs, les agents antisaignement, les agents actifs sur la gencive et les agents de polissage. Ces différents agents sont présents dans la composition selon l'invention notamment selon la forme qu'elle prend.

Ainsi, lorsque la composition à usage buccal est un spray, le véhicule peut être une solution hydro-alcoolique et la composition peut contenir en outre des arômes, des agents peptisants, des agents édulcorants, humectants ou rafraîchissants.

De même, à titre d'exemple, lorsque la composition est sous forme de bains de bouche, le véhicule peut être essentiellement constitué par une solution aqueuse d'un agent tensio-actif moussant, éventuellement avec un agent épaississant et peut comporter en outre des agents bactéricides, des édulcorants et des substances aromatisantes.

En outre, lorsque les compositions sont sous forme de gargarismes, elles peuvent renfermer un agent actif du type antibiotique.

A titre d'exemple, lorsque la composition est sous forme de gel dentaire, elle peut en outre contenir des agents actifs sur la gencive.

Dans la forme de réalisation des compositions sous forme de poudre, comprimés et granulés, les compositions comprennent en général essentiellement un agent tensio-actif moussant et un agent de polissage, l'agent de polissage étant présent dans des proportions pouvant aller jusqu'à 90 à 95% en poids.

Lorsque les compositions sont sous forme de gomme à mâcher, elles contiennent au moins une gomme masticable naturelle ou synthétique, et peuvent contenir des agents plastifiants, des vitamines, des agents d'aromatisation ou de sapidité, des agents sucrants, des agents humectants, des bactéricides, des conservateurs, des colorants et éventuellement des agents de polissage.

Parmi les gommes ayant une élasticité suffisante, seules ou en mélanges, pour être masticables, on peut citer parmi les gommes naturelles le latex d'Hévéa, la gomme chicle, la gomme schulong et parmi les gommes de synthèse, l'acétate de polyvinyle et les élastomères de synthèse : caoutchouc silicone, caoutchouc butyl, Généralement, ces gommes contiennent 0,5 à 70% en poids de gomme masticable.

Des agents de polissage peuvent être éventuellement utilisés selon l'invention. Pour les gommes à mâcher, on peut employer alors des agents de polissage usuels pour les gommes à mâcher, d'origine minérale ou organique. Ce sont par exemple les carbonate de calcium, de magnésium, de sodium, les phosphates et sulfates de calcium, l'alumine et l'alumine hydratée, les silices, les oxydes, hydroxydes, trisilicates et pyrophosphates de magnésium, ou des composés cellulosiques obtenus par broyage de graines de céréales.

Lorsque les compositions sont sous forme de pâte dentifrice, elles peuvent contenir un agent de polissage dans des proportions pouvant aller de 6 à 70% environ, et de préférence 15 à 25%. Il s'agit en général d'un matériau de polissage abrasif minéral constitué par un ou plusieurs composés, en grande partie insolubles dans l'eau. On peut citer par exemple les métaphosphates de sodium ou de potassium, le phosphate de calcium dihydraté, le phosphate dicalcique, le phosphate tricalcique, le pyrophosphate de calcium, l'alumine, les alumines hydratées et en particulier trihydratées, les silices, les silicates d'aluminium ou de zirconium, la bentonite ainsi que l'orthophosphate de magnésium ou le phosphate trimagnésien.

Lorsque la composition est sous forme de gel transparent, à titre d'agents de polissage, on peut utiliser un agent de polissage à base de silice colloïdale ou d'aluminosilicates de métaux alcalins ou alcalino-terreux, et de préférence de sodium ou de calcium.

De plus, dans certaines formes, et notamment dans les pâtes dentifrices les compositions selon l'invention peuvent contenir un ou plusieurs agents de cohésion. Ces agents de cohésion peuvent être incorporés dans des proportions pouvant aller jusqu'à 10% en poids par rapport au poids total de la composition, et de préférence de 0,5 à 3% en poids. Ceux-ci peuvent être choisis parmi les épaississants naturels tels que les alginates et les pectines, les gommes naturelles comme la gomme adragante, les gommes de xanthane, les gommes de guar, les gommes de caroube ou de carraghénanes ou des épaississants synthétiques, en général dérivés de cellulose comme le sel de sodium de la carboxyméthylcellulose, la méthylcellulose ou les hydroxyalkylcelluloses ou des acides polyacryliques réticulés comme les "CARBOPOLS".

Dans les compositions à usage buccal de l'invention, on peut utiliser en outre un agent édulcorant. Parmi les agents édulcorants utilisables, on peut citer le saccharose, le lactose, le fructose, le xylitol, le cyclamate de sodium, le maltose ou le saccharinate de sodium, les mélanges d'α-glucosyl/steviolglucoside. le D-Mannitol, l'Aspartame, l'Acesulfam K et leurs mélanges.

Ces agents édulcorants sont alors présents dans des concentrations allant jusqu'à 2%.

Comme agents humectants, on peut citer le sorbitol, le glycérol ou le xylitol, présents à ce titre dans des concentrations pouvant atteindre 50%.

En outre, les agents rafraîchissants, comme le menthol ou l'éthyl-maltol peuvent être incorporés.

Dans les compositions selon l'invention, il est également possible d'utiliser des agents conservateurs pour assurer une bonne pureté bactériologique des formulations. Ceux-ci peuvent être choisis parmi le parahydroxybenzoate de méthyle, le parahydroxybenzoate de propyle, entre autres agents conservateurs usuels. Les concentrations peuvent alors aller jusqu'à 0,5% en poids.

Comme substance aromatisante pour les compositions selon l'invention, on peut citer les essences de menthe, d'anis, d'eucalyptus, de cannelle, de girofle, de sauge, de réglisse, ou de fruits comme le citron, l'orange, la mandarine ou la fraise; on peut éventuellement utiliser à ce titre le salicylate de méthyle. Les substances aromatisantes, quand elles sont utilisées, peuvent l'être jusqu'à 5% en poids de la composition.

Lorsque l'on utilise un agent antibactérien, comme il est usuel d'en utiliser dans ce type de composition à usage buccal, on choisit de préférence des agents actifs dont certains sont des huiles essentielles ou des substances telles que la chlorhexidine, l'alexidine, l'octinidine, l'héxétidine, le phénoxyéthanol, l'alcool phénéthylique et le triclosan.

Lorsqu'ils sont présents dans les compositions, les agents bactériens peuvent représenter jusqu'à 10% en poids de la composition et sont présents de préférence à des concentrations comprises entre 0,05 et 2% en poids.

Enfin, il est possible d'incorporer dans les compositions à usage buccal selon l'invention, des agents anticaries comme le monofluorophosphate de sodium, les fluorures de sodium ou d'étain, les amines fluorées, les fluorures de polymère cationique tels que ceux qui sont décrits dans le brevet français 2 647 012.

Enfin, les compositions peuvent contenir des agents actifs tels que des agents antitartre, des agents cicatrisants, des agents vasomoteurs ou des agents antisaignement.

L'invention concerne donc l'utilisation d'une composition comportant un polymère défini ci-dessus et un véhicule physiologiquement acceptable pour son application à l'hygiène buccale et notamment à la prévention de la formation de taches sur les dents, de l'adhésion sur les dents des substances exogènes et de la plaque dentaire.

L'invention concerne également un procédé d'hygiène buccale, caractérisé en ce qu'on applique dans la cavité buccale une composition comportant au moins un polymère hydrocarboné, à structure linéaire ou réticulée, constitué principalement de motifs de formule : 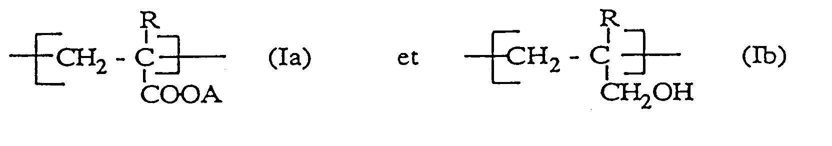 dans lesquelles :
R représente H ou CH₂OH,
A représente un métal alcalin ou un groupement sel d'ammonium, d'amine ou d'alcanolamine, la fréquence moyenne des motifs correspondant à un rapport carboxylate/hydroxyméthyle supérieur à 0,5 dans le polymère, et
   un véhicule physiologiquement acceptable.

L'invention concerne également un procédé de protection des dents naturelles ou artificielles par prévention de la formation de taches sur les dents, par protection des dents contre l'adhésion de substances exogènes et contre la formation de la plaque dentaire, le procédé étant mis en oeuvre par l'application des compositions de l'invention dans la cavité buccale.

L'application peut être réalisée par gargarisme, bain de bouche, dispersion, projection, mastication, brossage ou application avec le doigt ou avec un tampon sur la gencive et/ou les dents.

Les dénominations commerciales des produits cités dans la description et dans les exemples, notamment CARBOPOL, sont à la connaissance de la demanderesse, des marques déposées .

Les exemples présentés ci-après illustrent l'invention.

### EXEMPLE 1

On prépare une pâte dentifrice de composition suivante :

| | |
|---|---|
| - Silice précipitée amorphe, vendue sous la dénomination TIXOSIL 73 par la Société RHONE POULENC | 13 g |
| - Silice précipitée amorphe, vendue sous la dénomination TIXOSIL 43 par la Société RHONE POULENC | 7 g |
| - Dioxyde de titane | 0,5 g |
| - Carboxyméthylcellulose de sodium, vendue sous la dénomination BLANOSE 9M 31 S par la Société HERCULES | 1,4 g |
| - Sorbitol en solution aqueuse à 70% de MA | 22,4 g MA |
| - Laurylsulfate de sodium en poudre, vendu sous la dénomination EMPICOL LZV/E par la Société MARCHON à 93% de MA | 1,63 g MA |
| - Fluorure de sodium | 0,11 g |
| - Monofluorophosphate de sodium | 0,38 g |
| - Copolymère acrylate de sodium/vinylcarbinol de PM 7000-11000, vendu sous la dénomination HYDAGEN FN par la Société HENKEL | 4 g |
| - Agent édulcorant : Aspartame | 0,2 g |
| - Conservateur qs | |
| - Arôme qs | |
| - Eau qsp | 100 g |

### EXEMPLE 2

On prépare une pâte dentifrice de composition suivante :

| | |
|---|---|
| - Alumine hydratée vendue sous la dénomination ALUMINE SH 100 par la Société SOCHALU | 45 g |
| - Dioxyde de titane | 1 g |
| - Gomme de xanthane vendue sous la dénomination RHODOPOL 23 SC par la Société RHONE POULENC | 1,2 g |
| - Sorbitol en solution aqueuse à 70% de MA | 7 g MA |
| - Glycérine | 10 g |
| - Laurylsulfate de sodium en poudre, vendu sous la dénomination EMPICOL LZV/E par la Société MARCHON à 93% de MA | 1,86 g MA |
| - Fluorure de sodium | 0,22 g |
| - Saccharinate de sodium | 0,2 g |
| - Copolymère acrylate de sodium/vinylcarbinol de PM 7000-11000, vendu sous la dénomination HYDAGEN FN par la Société HENKEL | 1,25 g |
| - Arôme, conservateur qs | |
| - Eau qsp | 100 g |

### EXEMPLE 3

On prépare un gel dentifrice transparent de composition suivante :

| | |
|---|---|
| - Silice précipitée vendue sous la dénomination SIDENT 22 S par la Société DEGUSSA | 8 g |
| - Silice précipitée vendue sous la dénomination SIDENT 9 par la Société DEGUSSA | 12 g |
| - Carboxyméthylcellulose de sodium, vendue sous la dénomination BLANOSE 12M 31 XP par la Société HERCULES | 0,8 g |
| - Sorbitol en solution aqueuse à 70% de MA | 44,8 g MA |
| - Laurylsulfate de sodium en poudre, vendu sous la dénomination EMPICOL LZV/E par la Société MARCHON à 93% de MA | 1,4 g MA |
| - Saccharinate de sodium | 0,15 g |
| - Copolymère acrylate de sodium/vinylcarbinol de PM 7000-11000, vendu sous la dénomination HYDAGEN FN par la Société HENKEL | 0,4 g |
| - Polyéthylèneglycol à 8 moles d'oxyde d'éthylène | 2 g |
| - Arôme, conservateur, colorant qs | |
| - Eau qsp | 100 g |

### EXEMPLE 4

On prépare un spray buccal de composition suivante :

| | |
|---|---|
| - Alcool éthylique | 42 g |
| - Glycérine | 8 g |
| - Saccharinate de sodium | 0,05 g |
| - Copolymère acrylate de sodium/vinylcarbinol de PM 7000-11000, vendu sous la dénomination HYDAGEN FN par la Société HENKEL | 0,25 g |
| - Arôme | 1 g |
| - Peptisant | 2 g |
| - Eau qsp | 100 g |

### EXEMPLE 5

On prépare un bain de bouche de composition suivante :

| | |
|---|---|
| - Saccharinate de sodium | 0,02 g |
| - Phosphate trisodique | 0,04 g |
| - Phosphate monosodique | 0,08 g |
| - Alcool éthylique | 5 g |
| - Copolymère acrylate de sodium/vinylcarbinol de PM 7000-11000, vendu sous la dénomination HYDAGEN FN par la Société HENKEL | 0,5 g |
| - Sorbitol en solution aqueuse à 70% de MA | 3,5 g MA |
| - Arôme | 0,15 g |
| - Peptisant | 1,5 g |
| - Conservateur, colorant qs | |
| - Eau qsp | 100 g |

### EXEMPLE 6

On prépare un gel dentaire de composition suivante :

| | |
|---|---|
| - Acide polyacrylique réticulé, vendu sous la dénomination CARBOPOL 940 par la Société GOODRICH, neutralisé par NaOH | 0,5 g avant neutralisation |
| - Copolymère acrylate de sodium/vinylcarbinol de PM 7000-11000, vendu sous la dénomination HYDAGEN FN par la Société HENKEL | 0,1 g |
| - Sorbitol en solution aqueuse à 70% de MA | 7 g MA |
| - Saccharinate de sodium | 0,02 g |
| - Glycérine | 20 g |
| - Peptisant | 2 g |
| - Arôme | 0,1 g |
| - Conservateur qs | |
| - Eau qsp | 100 g |

### EXEMPLE 7

On prépare une gomme à mâcher de composition suivante :

| | |
|---|---|
| - Base élastique | 25 g |
| - Sorbitol en solution aqueuse à 70% de MA | 10,5 g MA |
| - Sorbitol en poudre | 53,4 g |
| - Copolymère acrylate de sodium/vinylcarbinol de PM 7000-11000, vendu sous la dénomination HYDAGEN FN par la Société HENKEL | 0,1 g |
| - Glycérine | 5 g |
| - Arôme | 1,5 g |

### EXEMPLE 8

On prépare une pâte dentifrice de composition suivante :

| | |
|---|---|
| - Silice précipitée amorphe, vendue sous la dénomination TIXOSIL 73 par la Société RHONE POULENC | 12 g |
| - Silice précipitée amorphe, vendue sous la dénomination TIXOSIL 333 par la Société RHONE POULENC | 8 g |
| - Laurylsulfate de sodium en poudre, vendu sous la dénomination EMPICOL LZV/E par la Société MARCHON à 93% de MA | 1,8 g MA |
| - Gomme de xanthane vendue sous la dénomination RHODOPOL 23SC par la Société RHONE POULENC | 1,2 g |
| - Dioxyde de titane | 0,6 g |
| - Sorbitol en solution aqueuse à 70% de MA | 30 g MA |
| - Parahydroxybenzoate de méthyle | 0,2 g |
| - Arôme qs | |
| - Polyéthylèneglycol à 8 moles d'oxyde d'éthylène | 1 g |
| - Copolymère acrylate de sodium/vinyl carbinol de PM 22500 ayant un degré de polymérisation compris entre 200 et 220 | 0,5 g |
| - Saccharinate de sodium | 0,2 g |
| - Eau qsp | 100 g |

## Revendications

1. Utilisation pour l'hygiène buccale d'une composition comportant au moins un polymère hydrocarboné à structure linéaire ou réticulée, constitué principalement de motifs de formule : 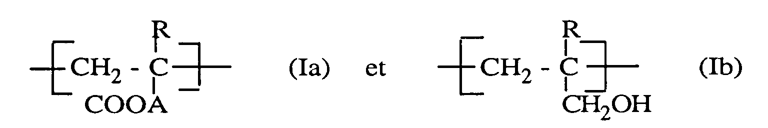 dans lesquelles :
R représente H ou CH₂OH,
A représente un métal alcalin ou un groupement sel d'ammonium, d'amine ou d'alcanolamine, la fréquence moyenne des motifs correspondant à un rapport carboxylate/hydroxyméthyle supérieur à 0,5 dans le polymère, et
un véhicule acceptable pour l'usage buccal.

2. Utilisation selon la revendication 1, caractérisée en ce que le degré de polymérisation moyen dudit polymère est compris entre 3 et 1000, et de préférence 50 à 600.

3. Utilisation selon l'une des revendications 1 ou 2, caractérisée en ce que la fréquence moyenne des motifs dans le polymère correspond à un rapport carboxylate/hydroxyméthyle compris entre 1,1 et 16.

4. Utilisation selon l'une des revendications 1 à 3, caractérisée en ce que dans la formule (Ia), A représente Na.

5. Utilisation selon l'une des revendications 1 à 4, caractérisée en ce qu'elle comporte 0,05 à 10% en poids de polymère par rapport au poids total de la composition.

6. Utilisation selon l'une des revendications 1 à 5, caractérisée en ce qu'elle comporte de 0,1 à 5% en poids de polymère par rapport au poids total de la composition.

7. Utilisation selon l'une des revendications 1 à 6, caractérisée en ce qu'elle est sous forme de gel ou pâte dentifrice, de bain de bouche, de mousse, de gargarisme, de poudre, de comprimés ou de granulés dentaires, de spray dentaire, de gel dentaire ou de gomme à mâcher.

8. Utilisation selon l'une des revendications 1 à 7, caractérisée en ce qu'elle comporte en outre un agent de polissage présent dans des proportions allant jusqu'à 95% en poids par rapport au poids total de la composition.

9. Utilisation selon l'une des revendications 1 à 8, caractérisée en ce qu'elle comporte jusqu'à 20% en poids par rapport au poids total de la composition de tensio-actifs de nature anionique, non-ionique, zwittérionique, amphotère ou cationique.

10. Utilisation selon l'une des revendications 1 à 9, caractérisée en ce qu'elle comporte au moins un agent de cohésion, un agent épaississant, un agent édulcorant, humectant ou rafraîchissant, un agent plastifiant, un arôme ou une substance ou un agent aromatisant, peptisant ou sucrant, un agent de sapidité, un agent conservateur, un agent antibactérien, un agent antibiotique, un agent anticaries, une vitamine, un agent antitartre, un colorant, un cicatrisant, un vasomoteur, un agent antisaignement, un agent actif sur la gencive, un agent actif inhibiteur de la mauvaise haleine.

11. Utilisation d'au moins un polymère hydrocarboné à structure linéaire ou réticulée, constitué principalement de motifs de formule : 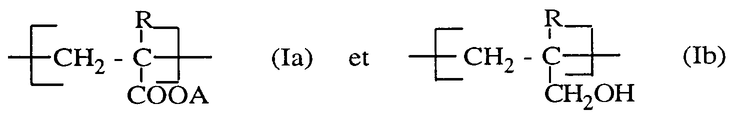 dans lesquelles :
R représente H ou CH₂OH,
A représente un métal alcalin ou un groupement sel d'ammonium, d'amine ou d'alcanolamine, la fréquence moyenne des motifs correspondant à un rapport carboxylate/hydroxyméthyle supérieur à 0,5 dans le polymère, pour la préparation d'une composition comportant ledit polymère et un véhicule physiologiquement acceptable, cette composition étant utile dans un traitement buccal.

12. Utilisation selon la revendication 11, caractérisée en ce que le traitement est cosmétique ou thérapeutique.

13. Procédé cosmétique de protection des dents naturelles ou artificielles contre les taches, l'adhésion des substances exogènes et la plaque dentaire, caractérisé en ce que l'on applique sur les dents et/ou les gencives, une quantité efficace d'une composition comportant au moins un polymère hydrocarboné à structure linéaire ou réticulée, constitué principalement de motifs de formule : 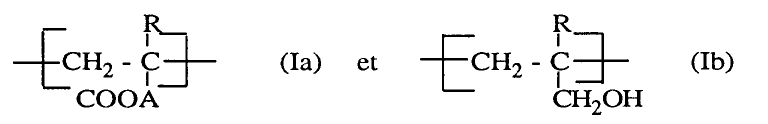 dans lesquelles :
R représente H ou CH₂OH,
A représente un métal alcalin ou un groupement sel d'ammonium, d'amine ou d'alcanolamine, la fréquence moyenne des motifs correspondant à un rapport carboxylate/hydroxyméthyle supérieur à 0,5 dans le polymère, et
un véhicule physiologiquement acceptable.

## Claims

1. Use for oral hygiene of a composition comprising at least one linear or crosslinked hydrocarbon polymer which is principally constituted by units with the following formula: 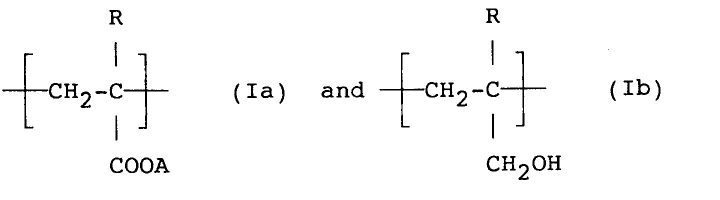 where:
R represents H or CH₂OH,
A represents an alkaline metal or an ammonium, amine or alkanolamine salt, the average frequency of the units corresponding to a carboxylate/hydroxymethyl ratio greater than 0.5 in the polymer, and
a physiologically acceptable vehicle.

2. Use according to claim 1 characterized in that the average degree of polymerization of said polymer is between 3 and 1 000, preferably 50 to 600.

3. Use according to claim 1 or claim 2 characterized in that the average frequency of the units in the polymer corresponds to a carboxylate/hydroxymethyl ratio of between 1.1 and 16.

4. Use according to any one of claims 1 to 3 characterized in that A represents Na in formula (Ia).

5. Use according to any one of claims 1 to 4 characterized in that it contains 0.05% to 10% by weight of polymer with respect to the total composition weight.

6. Use according to any one of claims 1 to 5 characterized in that it contains 0.1% to 5% by weight of polymer with respect to the total composition weight.

7. Use according to any one of claims 1 to 6 characterized in that it is in the form of a gel or toothpaste, mouthwash, foam, gargle, powder, dental tablet or granules, dental spray, dental gel or chewing gum.

8. Use according to any one of claims 1 to 7 characterized in that it further contains a polishing agent which is present in proportions of up to 95% by weight with respect to the total composition weight.

9. Use according to any one of claims 1 to 8 characterized in that it contains up to 20% by weight with respect to the total composition weight of anionic, non-ionic, zwitterionic, amphoteric or cationic surfactants.

10. Use according to any one of claims 1 to 9 characterized in that it contains at least one cohesive agent, thickener, sweetener, moistening agent, cooling agent, plasticizer, flavoring agent, aromatizing or peptizing agent or sweetener substance, palatabilizing agent, preservative, antibacterial agent, antibiotic agent, anticaries agent, vitamin, antitartar agent, dye, healing agent, vasomotor, clotting agent, active ingredient for gums, or active ingredient for inhibiting halitosis.

11. Use of a linear or crosslinked hydrocarbon polymer which is principally constituted by units with the following formula: 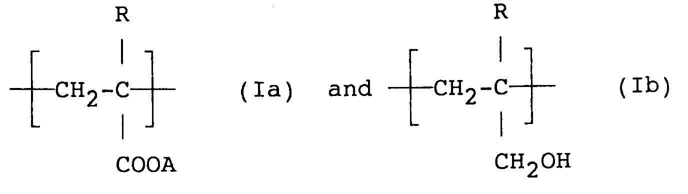 where:
R represents H or CH₂OH,
A represents an alkaline metal or an ammonium, amine or alkanolamine salt, the average frequency of the units corresponding to a carboxylate/hydroxymethyl ratio greater than 0.5 in the polymer, and a physiologically acceptable vehicle, this composition being useful for treatment of the mouth.

12. Use according to claim 11 characterized in that the treatment is cosmetic or therapeutic.

13. Cosmetic method of protecting natural or artificial teeth against stains, adhesion of exogenous substances and dental plaque characterized in that an effective quantity of a composition is applied to the teeth and/or the gums, the composition comprising at least one linear or crosslinked hydrocarbon polymer which is principally constituted by units with the following formula: 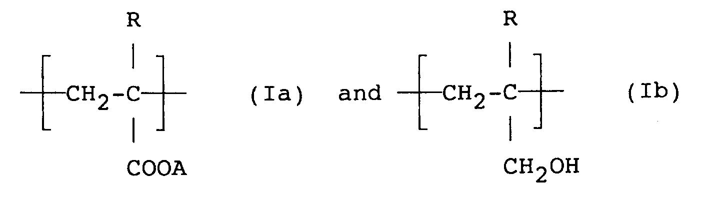 where:
R represents H or CH₂OH,
A represents an alkaline metal or an ammonium, amine or alkanolamine salt, the average frequency of the units corresponding to a carboxylate/hydroxymethyl ratio greater than 0.5 in the polymer, and
a physiologically acceptable vehicle.

## Patentansprüche

1. Zur Mundhygiene vorgesehene Verwendung einer Zusammensetzung, enthaltend mindestens ein Kohlenwasserstoffpolymer mit linearer oder vernetzter Struktur aus im wesentlichen Einheiten der Formel: 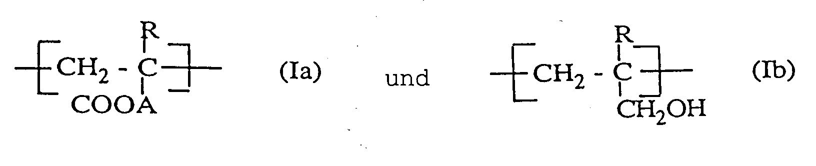 in denen gilt:
R stellt H oder CH₂OH dar,
A stellt ein Alkalimetall oder eine Ammonium-, Amin- oder Alkanolamin-Salzgruppierung dar, wobei die mittlere Häufigkeit der Einheiten einem Carboxylat/Hydroxymethyl-Verhältnis von mehr als 0,5 im Polymer entspricht, sowie
ein physiologisch geeignetes Trägermittel

2. Verwendung gemäß Anspruch 1,
dadurch **gekennzeichnet**, daß
der mittlere Polymerisationsgrad des genannten Polymer 3 bis 1000 und vorzugsweise 50 bis 600 beträgt.

3. Verwendung gemäß Anspruch 1 oder 2,
dadurch **gekennzeichnet**, daß
die mittlere Häufigkeit der Einheiten im Polymer einem Carboxylat-/Hydroxymethyl-Verhältnis von 1,1 bis 16 entspricht.

4. Verwendung gemäß einem der Ansprüche 1 bis 3,
dadurch **gekennzeichnet**, daß
in der Formel (Ia) A Na darstellt.

5. Verwendung gemäß einem der Ansprüche 1 bis 4,
dadurch **gekennzeichnet**, daß
die Zusammensetzung 0,05 bis 10 Gew.% Polymer, bezogen auf das Gesamtgewicht der Zusammensetzung, enthält.

6. Verwendung gemäß einem der Ansprüche 1 bis 5,
dadurch **gekennzeichnet**, daß
die Zusammensetzung 0,1 bis 5 Gew.% Polymer enthält, bezogen auf das Gesamtgewicht der Zusammensetzung.

7. Verwendung gemäß einem der Ansprüche 1 bis 6,
dadurch **gekennzeichnet**, daß
die Zusammensetzung in Form eines Zahngels oder einer Zahnpaste, eines Mundbads, Schaums, Gurgelmittels, Pulvers, in Form von Tabletten oder Körnern für die Zähne, eines Zahn-Sprühmittels, eines Gels für den Zahnbereich oder eines Kaugummi vorliegt.

8. Verwendung gemäß einem der Ansprüche 1 bis 7,
dadurch **gekennzeichnet**, daß
die Zusammensetzung ausserdem ein Poliermittel in Mengenanteilen bis zu 95 Gew.% enthält, bezogen auf das Gesamtgewicht der Zusammensetzung.

9. Verwendung gemäß einem der Ansprüche 1 bis 8,
dadurch **gekennzeichnet**, daß
die Zusammensetzung bis zu 20 Gew.%, bezogen auf das Gesamtgewicht der Zusammensetzung, oberflächenaktive Mittel anionischer, nicht-ionischer, zwitterionischer, amphoterer oder kationischer Art enthält.

10. Verwendung gemäß einem der Ansprüche 1 bis 9,
dadurch **gekennzeichnet**, daß
die Zusammensetzung mindestens ein Kohäsionsmittel, Verdickungsmittel, Süßungsmittel, Feuchtigkeits- oder Erfrischungsmittel, Plastifiziermittel, einen Aromastoff oder eine aromatisierende, peptisierende oder zuckernde Substanz, ein Geschmacksmittel, Konservierungsmittel, antibakterielles Mittel, antibiotisches Mittel, Antikaries-Mittel, ein Vitamin, ein Antizahnsteinmittel, einen Farbstoff, ein Vernarbungsmittel, vasomotorisches Mittel, Antiblutungsmittel, ein für das Zahnfleisch wirksames Mittel sowie ein zur Inhibierung von schlechtem Atem wirksames Mittel enthält.

11. Verwendung mindestens eines Kohlenwasserstoffpolymer mit linearer oder vernetzter Struktur aus im wesentlichen Einheiten der Formel: 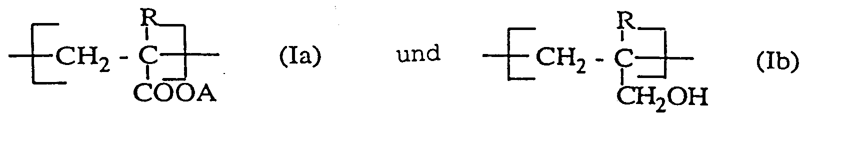 in denen gilt:
R stellt H oder CH₂OH dar,
A stellt ein Alkalimetall oder eine Ammonium-, Amin- oder Alkanolamin-Salzgruppierung dar, wobei die mittlere Häufigkeit der Einheiten einem Carboxylat/Hydroxymethyl-Verhältnis von mehr als 0,5 im Polymer entspricht, zur Herstellung einer Zusammensetzung, die das genannte Polymer und ein physiologisch geeignetes Trägermittel enthält, wobei sich diese Zusammensetzung zur Mundbehandlung eignet.

12. Verwendung gemäß Anspruch 11,
dadurch **gekennzeichnet**, daß
die Behandlung kosmetisch oder therapeutisch ist.

13. Kosmetisches Verfahren zum Schutz natürlicher oder künstlicher Zähne vor Flecken, Anhaftung exogener Substanzen und vor Zahnplaque,
dadurch **gekennzeichnet**, daß
man auf die Zähne und/oder das Zahnfleisch eine wirksame Menge einer Zusammensetzung aufbringt, die mindestens ein Kohlenwasserstoffpolymer mit linearer oder vernetzter Struktur aus im wesentlichen Einheiten der Formel: 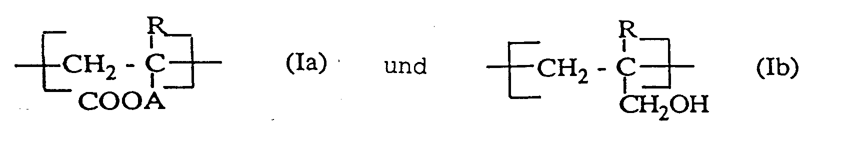 in denen gilt:
R stellt H oder CH₂OH dar,
A stellt ein Alkalimetall oder eine Ammonium-, Amin- oder Alkanolamin-Salzgruppierung dar, wobei die mittlere Häufigkeit der Einheiten einem Carboxylat/Hydroxymethyl-Verhältnis von mehr als 0,5 im Polymer entspricht, sowie
ein physiologisch geeignetes Trägermittel enthält.
